# DEMANDE DE BREVET EUROPEEN

(11) **EP 3 782 729 A1**
(43) Date de publication de la demande: **24.02.2021**
(21) Numéro de dépôt: 20191788.7
(22) Date de dépôt: 19.08.2020
(51) Int. Cl.: B01L 3/00, C12M 1/12, A61M 39/18, F16L 23/22

(54) **APPAREIL POUR MISE EN OEUVRE D'UN PROCÉDÉ DE TRAITEMENT DE FLUIDE DANS DES CONDITIONS STÉRILES**

(30) Priorité: 23.08.2019 FR 1909360
(71) Demandeur: Commissariat à l'Energie Atomique et aux Energies Alternatives, 75015 Paris (FR)
(72) Inventeur: RIVERA, Florence, 38054 GRENOBLE Cedex 09 (FR); BOTTAUSCI, Frédéric, 38054 GRENOBLE Cedex 09 (FR); GOUJON, Charles-Elie, 38054 GRENOBLE Cedex 09 (FR); PESCE, Jean-Luc, 38054 GRENOBLE Cedex 09 (FR); SARRUT-RIO, Nicolas, 38054 GRENOBLE Cedex 09 (FR)
(74) Mandataire: INNOV-GROUP

(57) **Abrégé**

L'invention concerne un appareil (1) automatisé employé dans la mise en œuvre d'un procédé de traitement d'un fluide dans des conditions stériles, comprenant :
- Un châssis (10) de support,
- Un module fluidique (2) fixé au châssis, comportant :
∘ un support fluidique (20) universel,
∘ une carte fluidique (21) dédiée amovible, fixée sur ledit support,
∘ un réservoir fluidique (22) d'échantillon qui est amovible et destiné à recevoir l'échantillon à traiter, connecté sur une entrée fluidique (214) spécifique du module fluidique (2),

- Plusieurs compartiments agencés sur le châssis (10) et comprenant chacun un réservoir (30) amovible, chaque réservoir (30) étant destiné à être relié à une entrée/sortie fluidique d'une liaison fluidique du support fluidique (20),
- Un système de commande intégré au châssis (10) et configuré pour assurer une circulation de fluides dans le module fluidique.

## Description

### Domaine technique de l'invention

La présente invention se rapporte à un appareil employé dans un procédé de traitement de fluide dans des conditions stériles et à un procédé de traitement de fluide dans des conditions stériles, mis en œuvre en employant ledit appareil.

### Etat de la technique

Il est connu que certains procédés doivent être mis en œuvre dans des conditions parfaitement stériles. C'est le cas par exemple d'un procédé destiné à réaliser une encapsulation de cellules. Dans ce procédé, on utilise un circuit micro-fluidique comprenant notamment deux canaux, un canal d'appauvrissement et un canal d'enrichissement juxtaposés sur un substrat et dans lesquels sont destinées à circuler deux phases liquides respectivement à appauvrir en éléments et à enrichir en éléments. La demande de brevet référencée WO2010/146261 A1 décrit notamment ce procédé et les moyens de le mettre œuvre.

Il existe par ailleurs des appareils de traitement de fluide dans des conditions stériles, notamment des appareils de culture cellulaire tels que ceux décrits dans les demandes de brevet EP2468844A1 et WO2018015561 A1**.**

La demande de brevet US2017/058243A1 décrit pour sa part un dispositif micro-fluidique de perfusion de cellules.

Dans la plupart des appareils connus, toutes les étapes du procédé doivent être réalisées sous un poste de sécurité microbiologique (appelé PSM), ce qui le rend plus difficile à mettre en œuvre, nécessitant forcément l'intervention d'un personnel qualifié, plus long et plus coûteux.

Le but de l'invention est donc de proposer un appareil permettant de limiter au maximum le nombre d'étapes à effectuer sous poste de sécurité microbiologique, dans le traitement d'un fluide nécessitant des conditions stériles. Cet appareil doit être particulièrement compact, simple à utiliser, fiable et facilement configurable selon le procédé de traitement mis en œuvre.

### Exposé de l'invention

Ce but est atteint par un appareil automatisé employé dans la mise en œuvre d'un procédé de traitement d'un fluide dans des conditions stériles, comprenant :
- Un châssis de support,
- Un module fluidique fixé au châssis, comportant :
   ∘ un support fluidique universel comprenant plusieurs liaisons fluidiques reliant chacune un premier point de connexion fluidique à une entrée/sortie fluidique,
   ∘ une carte fluidique dédiée amovible, fixée sur ledit support et comprenant plusieurs deuxièmes points de connexion fluidiques connectées chacun à un premier point de connexion fluidique distinct du support, un circuit fluidique ménagé entre ses points de connexion fluidiques, et plusieurs points de commandes pneumatiques,
   ∘ un réservoir fluidique d'échantillon qui est amovible et destiné à recevoir l'échantillon à traiter, connecté sur une entrée fluidique spécifique du module fluidique,
- Plusieurs compartiments agencés sur le châssis et comprenant chacun un réservoir amovible, chaque réservoir étant destiné à être relié à une entrée/sortie fluidique d'une liaison fluidique du support fluidique,
- Un système de commande intégré au châssis et configuré pour assurer une circulation de fluides dans le module fluidique,
- Caractérisé en ce que chaque entrée/sortie fluidique des liaisons fluidiques du support est muni d'un connecteur de type aseptique pour se connecter à chaque réservoir.

Selon une particularité, l'appareil comporte un bloc thermique fixé au châssis et comprenant un élément chauffant ou refroidissant et une unité associée de gestion dudit élément chauffant ou refroidissant.

Selon une autre particularité, l'appareil comporte un bloc de pressurisation fixé au châssis et configuré pour réaliser une mise en pression ou en dépression des fluides contenus dans lesdits réservoirs.

Selon une autre particularité, l'appareil comporte un bloc pneumatique auquel sont raccordés directement les points de commandes pneumatiques de la carte.

Selon une autre particularité, l'appareil comporte un bloc pneumatique auquel sont raccordés des points de commandes pneumatiques du support fluidique, ces points de commandes pneumatiques du support fluidique étant reliés auxdits points de commandes pneumatiques de la carte.

Selon une autre particularité, l'appareil comporte un bloc de ventilation fixé au châssis et comprenant au moins un ventilateur et une unité de gestion et de commande de ce ventilateur, tenant compte de la température présente à l'intérieur de l'appareil.

Selon une autre particularité, l'appareil comporte un logement spécifique, destiné à recevoir une poche stérile de collecte d'un fluide traité par la mise en œuvre du procédé.

Selon une autre particularité, l'appareil comporte un bloc de capture d'images, fixé au châssis et agencé au-dessus du module fluidique.

Selon une autre particularité, l'appareil comporte une unité de commande et de traitement.

L'invention concerne un procédé de traitement d'un fluide dans des conditions stériles, mis en œuvre à l'aide de l'appareil automatisé défini ci-dessus, le procédé comportant les étapes suivantes :
- Une étape de préparation du module fluidique, réalisée sur un poste de sécurité microbiologique et comprenant une fixation de ladite carte fluidique sur le support en connectant ses deuxièmes points de connexion fluidiques aux premiers points de connexion fluidiques du support, une connexion dudit réservoir d'échantillon dans lequel est placé un échantillon fluidique à traiter, sur l'entrée fluidique dédiée du module fluidique,
- Une étape de fixation du module fluidique sur l'appareil automatisé d'encapsulation et de connexion dudit module en raccordant chaque connecteur aseptique à un réservoir distinct de l'appareil et lesdits points de commandes pneumatiques de la carte à un bloc de commande pneumatique du système de commande,
- Une étape de commande dudit appareil via une unité de commande et de traitement.

Selon une autre particularité, il s'agit d'un procédé d'encapsulation de cellules.

### Brève description des figures

D'autres caractéristiques et avantages vont apparaître dans la description détaillée qui suit, faite en liaison avec les dessins annexés listés ci-dessous :
- Les figures 1A à 1C représentent, de manière schématique, l'architecture de l'appareil automatisé conforme à l'invention, selon trois variantes de réalisation.
- Les figures 2A et 2B représentent, en vue de dessus, le module fluidique employé dans l'appareil automatisé, respectivement pour la variante de réalisation de la figure 1A et pour celle de la figure 1B.
- La figure 3 illustre les différentes phases du procédé de l'invention, avec la réalisation de la figure 1A.
- La figure 4 représente un principe employé pour l'encapsulation de cellules.

### Description détaillée d'au moins un mode de réalisation

L'invention concerne notamment un appareil 1 automatisé permettant le traitement d'un fluide dans des conditions stériles. Nous parlons de traitement de fluide de manière générale, le type de traitement effectué étant permis selon le circuit fluidique présent dans la carte 21 et les étapes de commande mises en œuvre dans l'appareil.

A titre d'exemple et de manière non limitative, le traitement de fluide peut regrouper des méthodes d'encapsulation de cellules dans un liquide, de tri de cellules dans un liquide, de tri de bactéries présentes dans un liquide, de mélange de réactifs, de préparation de médicaments.

Par le terme "amovible" employé dans la description, on entend que l'élément concerné peut être facilement enlevé, en employant des outils classiques et/ou des moyens intégrés tels que clipsage, emboîtement, vissage...

L'appareil 1 se présente sous la forme d'un ensemble monobloc comprenant un châssis 10. Ce châssis 10 supporte plusieurs ensembles nécessaires au fonctionnement de l'appareil. On verra que seule l'unité de commande et de traitement UC peut être fixée ou séparée par rapport au châssis 10 et aux ensembles montés sur ce châssis. L'appareil 1 peut ensuite comporter un boîtier réalisé en un ou plusieurs éléments, par exemple en matériau plastique et formant le carénage de l'appareil.

L'appareil 1 comporte un module fluidique 2 fixé au châssis 10. Ce module fluidique 2 est amovible par rapport au châssis.

Le module fluidique 2 est avantageusement composé d'au moins trois éléments distincts. Il comporte en effet un support fluidique 20 universel fixé de manière amovible au châssis, une carte fluidique 21 fixée de manière amovible et uniquement au support fluidique et un réservoir fluidique 22 destiné à être connecté directement sur la carte 21 ou sur le support.

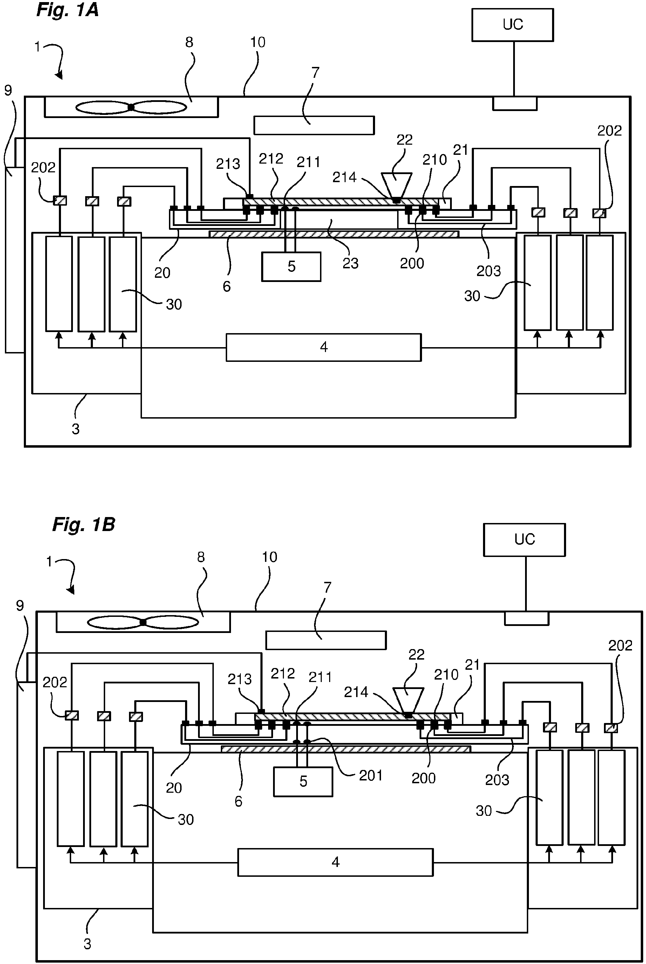

Le support fluidique 20 se présente sous la forme d'une plaque standard intégrant un circuit fluidique doté de plusieurs liaisons fluidiques 203 distinctes reliant chacune un point de connexion fluidique 200, dit premier point de connexion fluidique, accessible sur une face du support (face supérieure ou inférieure selon la configuration) à une entrée/sortie fluidique connectable sur un réservoir de fluide distinct. Dans une configuration particulière représentée sur la figure 1B, il peut également intégrer un circuit pneumatique comprenant plusieurs points de commandes pneumatiques, dits premiers points de commandes pneumatiques 201.

Chaque entrée/sortie fluidique du support 20 est munie d'un connecteur aseptique 202 permettant le raccord entre chaque liaison fluidique 203 et un réservoir de fluide 30 distinct. Ces connecteurs 202 permettent de conserver un module fluidique 2 parfaitement stérile jusqu'à sa mise en place dans l'appareil 1 et la connexion des liaisons fluidiques 203 du support 20 aux réservoirs 30 de liquides présents dans l'appareil 1, puis après sa mise en place dans l'appareil 1 et tout au long de la mise en œ uvre du procédé. Les connecteurs aseptiques 202 sont de type mâle/femelle, par exemple de type "Opta SFT" ("Sartorius Stedim"), ou "Aseptic Quick" ("Aseptic Process").

Le support fluidique 20 est universel et peut accueillir différentes cartes 21 fluidiques, se différenciant chacune d'elles par leur circuit fluidique. Le support 20 comporte deux faces principales opposées, une face dite supérieure et une face dite inférieure.

La carte fluidique 21 est destinée à venir en appui mécaniquement contre une face du support 20, face supérieure ou face inférieure selon la configuration choisie (figures 1A à 1C). Elle comporte pour sa part plusieurs points de connexion fluidiques 210, dits deuxièmes points de connexion fluidiques, destinés chacun à se connecter à un premier point de connexion fluidique 200 distinct du support 20 par appui mécanique contre le support 20 et des points de commandes pneumatiques, dits deuxièmes points de commandes pneumatiques 211. Chaque point de connexion fluidique de la carte forme une entrée/sortie fluidique du circuit fluidique intégré à la carte 21.

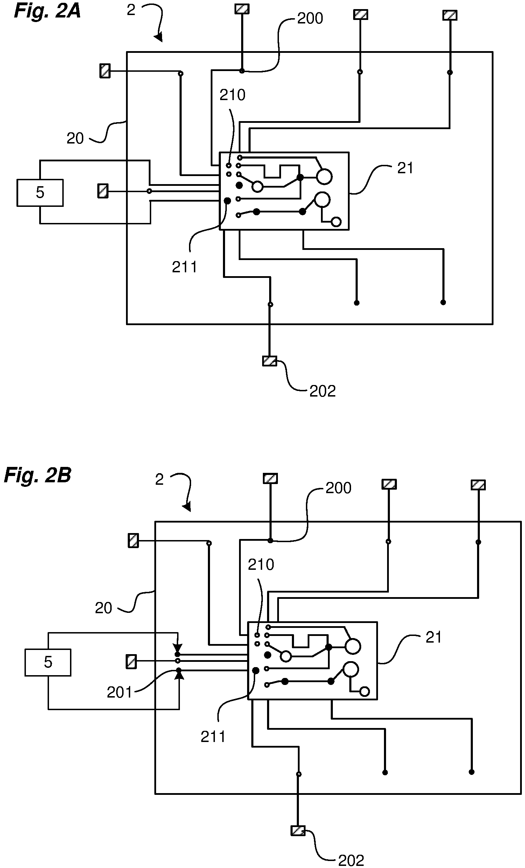

Dans une première variante de réalisation représentée sur les figures 1A et 2A, ses deuxièmes points de commandes pneumatiques 211 sont raccordés directement au bloc pneumatique 5 décrit ci-dessous. Le support fluidique 20 peut comporter une ouverture 23 traversée par les liaisons pneumatiques reliant directement le bloc pneumatique 5 aux points de commandes pneumatiques 211 présents sur la carte 21.

Dans une deuxième variante de réalisation représentée sur les figures 1B et 2B, les deuxièmes points de commandes pneumatiques 211 sont raccordés auxdits premiers points de commandes pneumatiques 201 présents sur le support 20 et déjà évoqués ci-dessus. Et ces premiers points de commandes pneumatiques 201 sont raccordés au bloc pneumatique 5.

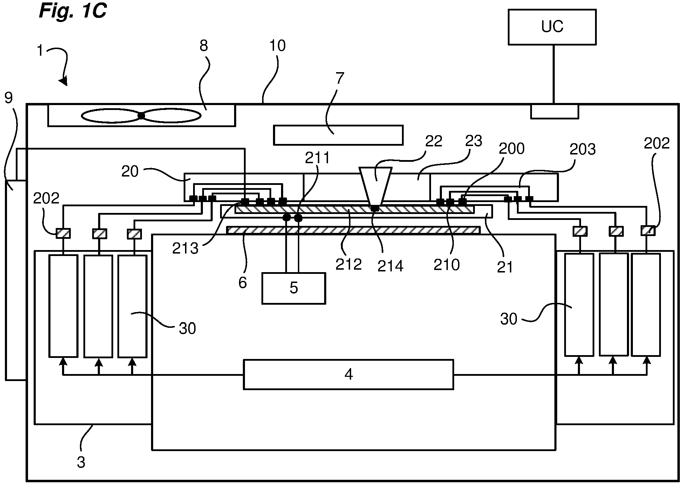

Dans les deux réalisations représentées sur les figures 1A et 1B, la carte fluidique 21 est apposée sur la face supérieure du support 20. Dans la réalisation de la figure 1C, la carte 21 est apposée contre la face inférieure du support 20.

Les deuxièmes points de connexion fluidiques 210 de la carte 21 sont donc agencés selon la réalisation envisagée. Ils seront accessibles sur la face inférieure de la carte 21 dans les réalisations des figures 1A et 1B et sur la face supérieure de la carte 21 dans la réalisation de la figure 1C.

Il faut comprendre que ces deux configurations (montage de la carte par le dessus ou montage par le dessous) sont équivalentes et que l'ensemble des caractéristiques de l'invention s'applique quelle que soit la configuration qui est adoptée.

Les connexions fluidiques entre la carte 21 et le support 20 sont avantageusement réalisées en mettant en vis-à-vis les points de connexion respectifs des deux éléments et en exerçant une pression mécanique suffisante de la carte 21 contre le support 20, afin d'obtenir des connexions fluidiques étanches.

La carte fluidique 21 est dite propriétaire (par opposition à universel pour le support fluidique), c'est-à-dire que, entre ses différentes entrées/sorties fluidiques, elle comporte un circuit fluidique 212 qui est adapté au procédé de traitement de fluide à mettre en œuvre. Il peut notamment s'agir d'un circuit fluidique 212 permettant de réaliser une encapsulation de cellules, tel que décrit dans la demande de brevet référencée WO2010/146261 A1.

De manière non limitative, la carte fluidique 21 est composée d'un empilement de plaques plastiques (type COC, COP, ...) transparentes qui sont soient usinées, soient moulées et/ou percées, de manière à y former le circuit fluidique. Ces plaques contiennent des canaux ayant par exemple des sections d'écoulement rectangulaires présentant des dimensions de 500µm par 550µm allant jusqu'à 500µm par 800µm. Elles contiennent également des vannes (d'ouverture, de l'ordre de 800µm de diamètre) reliées aux points de commande pneumatique et de trous de connexion (de l'ordre de 800µm de diamètre) formant les entrées/sorties fluidiques du circuit. Ces plaques sont scellées thermiquement ou chimiquement afin d'assurer une étanchéité fluidique.

Un point de commande pneumatique est constitué d'une entrée pneumatique sur laquelle viennent se connecter, directement ou indirectement, des moyens permettant d'exercer une pression ou une dépression pour actionner une vanne ou tout autre élément à actionnement pneumatique du module fluidique.

Le réservoir fluidique 22 est configuré pour se connecter directement sur une entrée fluidique 214 de la carte fluidique 21 ou sur une entrée fluidique du support fluidique 20. Ce réservoir 22 peut se présenter sous la forme d'un cône disposant d'une connectique fluidique adaptée pour coopérer avec la connectique de l'entrée fluidique de la carte 21. Ce réservoir 22 est destiné à recevoir l'échantillon liquide ECH à traiter/analyser. Ce réservoir 22 peut venir se clipser ou se visser à la carte 21 ou au support 20. Il est ainsi positionné au plus proche du circuit fluidique de traitement, ce qui permet de minimiser les volumes morts et donc les pertes de cellules et de polymères dans le cas d'un procédé d'encapsulation de cellules.

L'appareil 1 comporte également plusieurs compartiments formés de racks 3 fixés au châssis 10 et destinés chacun à recevoir un réservoir 30 de fluide destiné au procédé mis en œuvre. L'appareil peut par exemple comporter six à huit compartiments distincts. Chaque réservoir 30 peut être formée d'une bouteille placée dans le compartiment. Chaque rack 3 peut être incliné de manière à pouvoir y insérer le réservoir.

Selon une particularité, chaque réservoir 30 présente une contenance particulièrement importante, étant d'au moins 1000 fois le volume de fluide traité dans la carte fluidique (c'est-à-dire présent dans le circuit fluidique 212 de la carte). L'utilisation de réservoirs 30 de très grande capacité permet de traiter de gros volumes de fluide et de faire fonctionner le procédé en continu, c'est-à-dire avec un minimum d'interruptions.

Pour faire fonctionner le module fluidique 2 et donc mettre en œuvre le procédé visé, l'appareil 1 comporte plusieurs autres blocs fixés sur son châssis 10 et logés dans son boîtier.

L'appareil comporte ainsi un bloc 4 de pressurisation, c'est-à-dire de mise en pression des fluides présents dans les réservoirs 30 placés dans les compartiments de l'appareil 1. Ce bloc 4 est fixé au châssis 10 et peut comporter des modules de pompage choisis avec des temps de réaction court afin de pouvoir répondre aux changements de pression inhérents au procédé mis en œuvre dans la carte.

L'appareil 1 comporte également un bloc pneumatique 5 fixé au châssis 10 et pouvant être composé d'un microcontrôleur, de cartes opto-relais et de blocs d'électrovannes. Ce bloc pneumatique 5 a pour objet d'actionner les vannes présentes sur la carte fluidique, via les points de commandes pneumatiques présents sur le support et/ou sur la carte (selon la configuration choisie). La connexion du bloc pneumatique avec les différents points de commandes pneumatiques du module fluidique est hermétique et peut être assurée par des joints toriques ou des membranes moulées en silicone.

L'appareil 1 peut comporter un module thermique 6 à effet chauffant ou refroidissant, composé d'une résistance ou d'un élément à effet Peltier. Ce module thermique 6 est fixé au châssis 10 et placé sous le module fluidique 2, préférentiellement sous la carte fluidique 21 pour chauffer celle-ci de manière localisée à une température souhaitée. Le module thermique 6 comporte une unité de gestion de la température configurée pour régler et réguler la température de chauffage à une valeur fixe souhaitée.

L'appareil 1 peut comporter un bloc de capture d'images 7, permettant de suivre le procédé mis en œuvre dans le circuit fluidique 212 de la carte 21. Ce bloc est fixé au châssis 10 et logé dans le boîtier de l'appareil. Il est par exemple positionné au-dessus du module fluidique et de la carte fluidique de manière à capturer des images du circuit fluidique. Ce bloc peut comporter une caméra déplaçable dans les trois axes de l'espace.

L'appareil peut ensuite comporter un bloc mécanique (non représenté) permettant d'assurer de manière fiable les connexions fluidiques et pneumatiques entre la carte fluidique et son support et entre le support et le bloc pneumatique de l'appareil.

L'appareil 1 peut également comporter un bloc de ventilation 8 comprenant au moins un ventilateur et une unité de gestion et de commande de ce ventilateur, tenant compte de la température présente à l'intérieur de l'appareil.

L'appareil peut également comporter un logement 9 spécifique, ménagé dans le boîtier de l'appareil 1 et destiné à recevoir une poche stérile de collecte d'un fluide traité par la mise en œuvre du procédé. Dans le cas d'un procédé d'encapsulation de cellules, cette poche est destinée à recevoir le liquide contenant les cellules encapsulées. Cette poche est reliée à une entrée/sortie fluidique 213 du module fluidique, présente sur la carte 21 (comme sur la figure 1) ou sur le support 20.

L'appareil 1 peut également comporter une unité de commande et de traitement UC. Cette unité de commande et de traitement UC peut être intégrée et logée dans le boîtier de l'appareil 1 ou être externe à celui-ci. Il peut s'agir d'un automate programmable comprenant plusieurs modules d'entrées/sorties sur lesquelles sont connectés les différents blocs décrits ci-dessus et un module de traitement chargé de dérouler le procédé de commande. L'unité de commande et de traitement UC est configurée pour exécuter les différentes étapes nécessaires à la mise en œuvre du procédé dans l'appareil, en émettant des commandes aux différents blocs de l'appareil 1. A titre d'exemple, lorsqu'une étape nécessite l'injection d'un fluide présent dans l'un des réservoirs de l'appareil 1, elle va envoyer une commande au bloc de pressurisation 4 comprenant la voie sur laquelle est connecté le réservoir 30 dont le liquide doit être mis en pression. Puis elle va envoyer une ou plusieurs commandes au bloc pneumatique 5 pour commander une ou plusieurs vannes du circuit fluidique 212 de la carte, selon le circuit à faire emprunter au fluide lors de la réalisation de cette étape.

L'appareil décrit ci-dessus permet de mettre en œuvre de manière simple un procédé de traitement d'un fluide, dans des conditions stériles, et en limitant les étapes effectuées sur un poste de sécurité microbiologique (appelé également PSM). Cela est notamment permis par l'appareil de l'invention, et notamment grâce à son module fluidique 2 amovible et à sa carte fluidique 21 interchangeable et adaptable au procédé mis en œuvre.

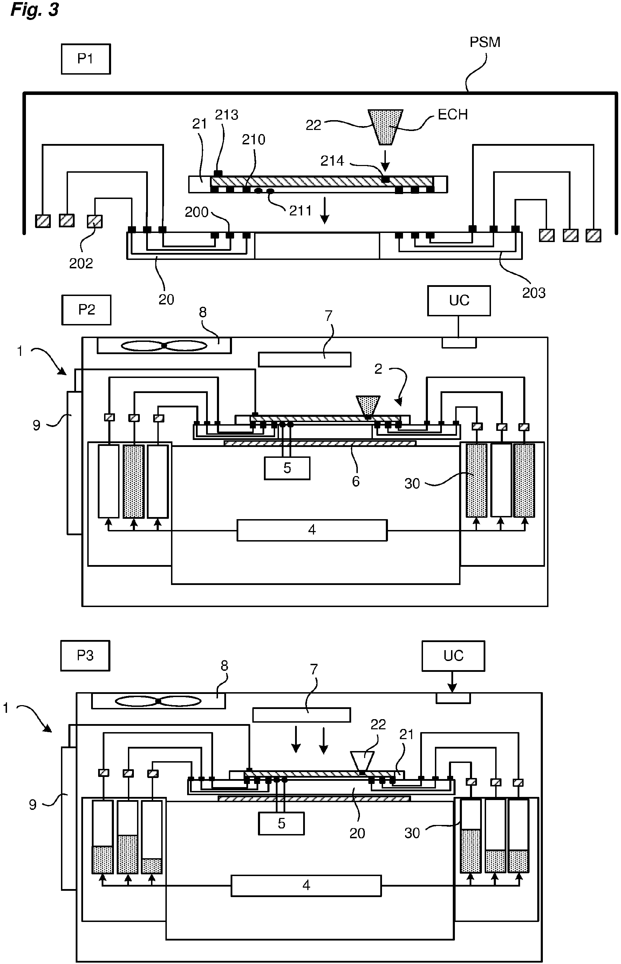

En référence à la figure 3, un procédé de traitement de fluide mis en œuvre en employant l'appareil 1 automatisé de l'invention tel que décrit ci-dessus, comporte les phases suivantes :
- Une phase P1 préalable de préparation du module fluidique 2, livré sous emballage stérile, cette phase étant réalisée sous un poste de sécurité microbiologique PSM et comprenant les étapes suivantes :
   ∘ Préparation de l'échantillon ECH, par exemple par mélange - dans le cas d'un procédé d'encapsulation de cellules, il s'agit d'un mélange cellules-polymères.
   ∘ Injection de l'échantillon ECH préparé dans le réservoir 22 et fermeture hermétique du réservoir.
   ∘ Connexion du réservoir 22 sur l'entrée fluidique spécifique du module fluidique 2 (cette entrée pouvant être sur le support ou directement sur la carte), via la connectique adaptée (type Luer, clipsage, vissage...).
   ∘ Connexion de la carte 21 sur le support fluidique 20, en connectant les points de connexion fluidiques 200 du support aux points de connexion fluidiques 210 de la carte et, selon la configuration du module, les points de commande pneumatique 211 de la carte directement au bloc pneumatique 5 (configuration de la figure 1A) ou les points de commande pneumatique 201 du support aux points de commande pneumatique 211 de la carte (ces derniers étant déjà reliés au bloc pneumatique 5 - configuration de la figure 1B). Des moyens d'alignement mécanique et/ou magnétique peuvent être prévus pour assurer le positionnement de la carte 21 sur le support 20. Les connexions fluidiques étanches entre la carte 21 et le support 20 peuvent être réalisées par pression mécanique de la carte 21 contre le support 20.
- Une deuxième phase P2 de préparation de l'appareil, pouvant être réalisée en dehors du poste de sécurité microbiologique PSM et comprenant les étapes suivantes :
   ∘ Fixation du module fluidique 2 sur le châssis 10 de l'appareil 1.
   ∘ Connexion des liaisons fluidiques 203 du support aux réservoirs 30 via les connecteurs aseptiques 202.
   ∘ Connexion de la sortie fluidique 213 à la poche de collecte.
   ∘ Allumage de l'unité de commande et de traitement UC.
   ∘ Sélection du procédé de traitement à effectuer et réglage (automatique et/ou manuel) des paramètres de fonctionnement du procédé sélectionné.
- Une troisième phase P3 d'exécution du procédé de traitement sélectionné, dans l'appareil 1, comprenant plusieurs étapes exécutées par l'unité de commande et de traitement UC. Chaque étape peut consister en des commandes du bloc de pressurisation 4, du bloc pneumatique 5, du bloc thermique 6, de surveillance des conditions de fonctionnement (température notamment).

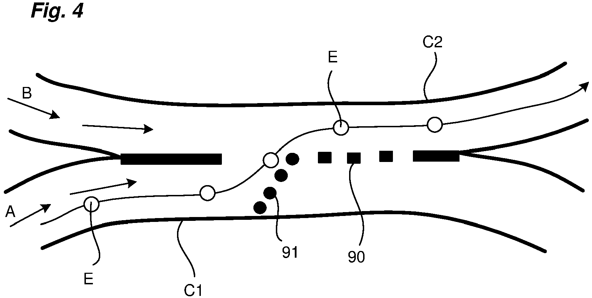

A titre d'exemple, en référence à la figure 4, lors d'un procédé d'encapsulation de cellules, le circuit fluidique 212 réalisé sur la carte 21 peut comporter deux canaux C1, C2, respectivement d'appauvrissement et d'enrichissement qui sont juxtaposés de manière sensiblement parallèle sur le substrat. Dans le canal d'appauvrissement C1 circule une phase liquide A à appauvrir en éléments E particuliers et dans le canal d'enrichissement C2 circule une phase liquide B à enrichir. Les deux phases liquides sont choisies immiscibles entre elles (par exemple huile et eau). Chaque canal C1, C2 présente une entrée et une sortie. Les deux canaux sont réunis entre eux via une zone de jonction, formant une zone d'échange permettant le contact entre les deux phases liquides. Par des moyens hydrodynamiques, des éléments E (tels que des cellules encapsulés) sont transférés d'une phase à l'autre. La zone de jonction peut comporter des piliers 90 de séparation. Des plots 91 de déviation peuvent être positionnés dans le canal C1 pour orienter les éléments E vers le canal C2.

L'appareil 1 de l'invention présente ainsi de nombreux avantages, parmi lesquels :
- Il permet de traiter des gros volumes de liquides, notamment grâce à l'emploi des réservoirs, déportés par rapport à la carte fluidique.
- Il permet de limiter le nombre d'étapes à effectuer sous poste de sécurité microbiologique, simplifiant le procédé global.
- Il permet de réaliser différents traitements de fluide (encapsulation de cellules, tri de cellules, tri de bactéries) en employant un même appareil, simplement en introduisant la carte fluidique adaptée et en sélectionnant le procédé de traitement associé à cette carte.
- Il peut être réalisé avec une architecture particulièrement compacte.

## Revendications

1. Appareil (1) automatisé employé dans la mise en œuvre d'un procédé de traitement d'un fluide dans des conditions stériles, comprenant :
- Un châssis (10) de support,
- Un module fluidique (2) fixé au châssis, comportant :
▪ un support fluidique (20) universel comprenant plusieurs liaisons fluidiques (203) reliant chacune un premier point de connexion fluidique (200) à une entrée/sortie fluidique,
▪ une carte fluidique (21) dédiée amovible, fixée sur ledit support et comprenant plusieurs deuxièmes points de connexion fluidiques (210) connectées chacun à un premier point de connexion fluidique (200) distinct du support, un circuit fluidique ménagé entre ses points de connexion fluidiques (210), et plusieurs points de commandes pneumatiques (211),
▪ un réservoir fluidique (22) d'échantillon qui est amovible et destiné à recevoir l'échantillon à traiter, connecté sur une entrée fluidique (214) spécifique du module fluidique (2),
- Plusieurs compartiments agencés sur le châssis (10) et comprenant chacun un réservoir (30) amovible, chaque réservoir (30) étant destiné à être relié à une entrée/sortie fluidique d'une liaison fluidique du support fluidique (20),
- Un système de commande intégré au châssis (10) et configuré pour assurer une circulation de fluides dans le module fluidique, **Caractérisé en ce que** chaque entrée/sortie fluidique des liaisons fluidiques (203) du support est muni d'un connecteur (202) de type aseptique pour se connecter à chaque réservoir (30).

2. Appareil selon la revendication 1, **caractérisé en ce qu'**il comporte un bloc thermique (6) fixé au châssis et comprenant un élément chauffant ou refroidissant et une unité associée de gestion dudit élément chauffant ou refroidissant.

3. Appareil selon la revendication 1 ou 2, **caractérisé en ce qu'**il comporte un bloc de pressurisation (4) fixé au châssis et configuré pour réaliser une mise en pression ou en dépression des fluides contenus dans lesdits réservoirs.

4. Appareil selon l'une des revendications 1 à 3, **caractérisé en ce qu'**il comporte un bloc pneumatique (5) auquel sont raccordés directement les points de commandes pneumatiques (211) de la carte (21).

5. Appareil selon l'une des revendications 1 à 3, **caractérisé en ce qu'**il comporte un bloc pneumatique (5) auquel sont raccordés des points de commandes pneumatiques (201) du support fluidique (20), ces points de commandes pneumatiques (201) du support fluidique (20) étant reliés auxdits points de commandes pneumatiques (211) de la carte (21).

6. Appareil selon l'une des revendications 1 à 5, **caractérisé en ce qu'**il comporte un bloc de ventilation (8) fixé au châssis (10) et comprenant au moins un ventilateur et une unité de gestion et de commande de ce ventilateur, tenant compte de la température présente à l'intérieur de l'appareil.

7. Appareil selon l'une des revendications 1 à 6, **caractérisé en ce qu'**il comporte un logement (9) spécifique, destiné à recevoir une poche stérile de collecte d'un fluide traité par la mise en œuvre du procédé.

8. Appareil selon l'une des revendications 1 à 7, **caractérisé en ce qu'**il comporte un bloc de capture d'images (7), fixé au châssis (10) et agencé au-dessus du module fluidique (2).

9. Appareil selon l'une des revendications 1 à 8, **caractérisé en ce qu'**il comporte une unité de commande et de traitement (UC).

10. Procédé de traitement d'un fluide dans des conditions stériles, mis en œuvre à l'aide de l'appareil (1) automatisé défini dans l'une des revendications 1 à 9, **caractérisé en ce qu'**il comporte les étapes suivantes :
- Une étape de préparation du module fluidique (2), réalisée sur un poste de sécurité microbiologique (PSM) et comprenant une fixation de ladite carte fluidique (21) sur le support (20) en connectant ses deuxièmes points de connexion fluidiques (210) aux premiers points de connexion fluidiques (200) du support, une connexion dudit réservoir (22) d'échantillon dans lequel est placé un échantillon fluidique à traiter, sur l'entrée fluidique (212) dédiée du module fluidique,
- Une étape de fixation du module fluidique (20) sur l'appareil (1) automatisé d'encapsulation et de connexion dudit module (20) en raccordant chaque connecteur aseptique (202) à un réservoir (30) distinct de l'appareil et lesdits points de commandes pneumatiques (211) de la carte à un bloc de commande pneumatique (5) du système de commande,
- Une étape de commande dudit appareil (1) via une unité de commande et de traitement (UC).

11. Procédé selon la revendication 10, **caractérisé en ce qu'**il s'agit d'un procédé d'encapsulation de cellules.
